(19) Europäisches Patentamt
European Patent Office
Office européen des brevets

(11) **EP 4 265 238 A1**

(12) **EUROPÄISCHE PATENTANMELDUNG**

(43) Veröffentlichungstag:
**25.10.2023  Patentblatt 2023/43**

(21) Anmeldenummer: **23154190.5**

(22) Anmeldetag: **31.01.2023**

(51) Internationale Patentklassifikation (IPC):
**A61K 8/81** *(2006.01)*     **A61Q 5/00** *(2006.01)*
**A61Q 5/06** *(2006.01)*

(52) Gemeinsame Patentklassifikation (CPC):
**A61K 8/8152; A61Q 5/00; A61Q 5/06**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **25.02.2022  DE 102022201995**

(71) Anmelder: **Beiersdorf AG**
**20245 Hamburg (DE)**

(72) Erfinder:
• **Saß, Viola**
 **22880 Wedel (DE)**
• **Hetzel, Frank**
 **21261 Welle (DE)**
• **Nemnich, Julia**
 **22589 Hamburg (DE)**

(74) Vertreter: **Beiersdorf AG**
**Patentabteilung**
**Unnastraße 48**
**20245 Hamburg (DE)**

(54) **HAAR-KOSMETISCHE ZUBEREITUNG, DIE EINEN UV-SCHUTZ BEWIRKT**

(57)    Verwendung einer festigenden Haar-kosmetischen Zubereitung, die ein Octylacrylamide Acrylates Butylaminoethyl Methacrylate Copolymer in einer alkoholischen Lösung enthält, zum UV-Schutz der Haare.

**EP 4 265 238 A1**

**Beschreibung**

Die vorliegende Erfindung beschreibt eine Haar-kosmetische Zubereitung, insbesondere ein Haarspray oder ein Pumpspray, zum Stylen und/oder Formen der Haare. Durch die Anwendung der genannten Zubereitung erhalten die Haare einen UV-Schutz.

Haare sind ein wichtiger Teil des menschlichen Körpers. Der ganze menschliche Körper mit Ausnahme der Lippen, der Handinnenflächen und der Fußsohlen, ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Zu den äußeren Einflüssen, die auf das Haar einwirken, ist auch UV-Licht zu zählen. UV-Licht ist ein Energiereiches Licht, das zum einen auf Farbpigmente einwirkt und sie zerstören kann. Dies gilt für die natürlichen Farbpigmente, die Melanine, genauso wie für Pigmente oder Farbstoffe, die mittels Haarfärbungen oder Haartönungen auf das Haar gelangen. Durch das UV-Licht wird die Farbe der Haare blasser, die Haare bleichen aus.

Zum anderen wirkt das UV-Licht auch auf die Proteinstrukturen der Haare ein und schädigt diese. Die Folge sind Haare mit Spliss und/oder Haare, die brüchig sind.

Darüber hinaus führt das UV-Licht auch dazu, dass die Haare ein stumpfes Aussehen bekommen.

Es ist also überaus wünschenswert, für die Haare einen UV-Schutz bereit zu stellen, um den genannten negativen Phänomenen entgegen zu wirken.

Natürliches Haar hat in den meisten Fällen wenig eigenes Volumen und bei langem Haar hängt es oftmals recht schlaff und gerade vom Kopf herab. Viele Menschen wünschen sich aber Haare, die mehr Volumen aufweisen und sich auf gefällige Art formen und/oder stylen lassen. Dies kann grundsätzlich auf zweierlei Weise erzielt werden, zum einen gibt permanente Haarverformungsverfahren, beispielsweise die Dauerwelle und nicht permanente Verfahren, die die Frisur des Haares nur für eine begrenzte Zeit formen und fixieren. Um eine nicht-permanente Frisurgestaltung zu erreichen, werden mehrere Produktformen zur Verfügung gestellt, die einzeln oder in Kombination miteinander verwendet werden können. Zu nennen seien hier beispielsweise Festiger, Schaumfestiger, Haarsprays, Haarwachse, Haargele und andere mehr. Es können Produktformen unterschieden werden, die vorzugsweise auf das nasse Haar aufgetragen und eingearbeitet werden und Produktformen, die auf das trockene Haar aufgetragen werden.

Bekannte Produktformen, die auf das trockene Haar aufgetragen werden können, sind Haargele, Haarwachse und Gelsprays, wobei letztere vielfach im asiatischen Raum zur Anwendung kommen. Die trockenen Haare werden vor oder nach Anwendung der genannten Produkte in die gewünschte Form gebracht, beispielsweise durch die Hände, mithilfe von Bürsten oder auf andere Weise.

Obwohl schon lange bekannt und seit langem käuflich zu erwerben, sind Haarsprays immer noch sehr beliebt bei den Verbraucherinnen. Sie sind einfach anzuwenden und erzielen in der Regel einen überaus befriedigen Halt der Frisur.

Haarsprays sind Zubereitungen, die ein festigendes und/oder Film-bildendes Polymer enthalten. Diese Art von Polymeren wechselwirken mit der Haaroberfläche und bilden einen Film aus. Zwischen einzelnen Haaren oder Haarsträhnen können dann kleine Brücken aus Polymeren ausgebildet werden, die wesentlich dazu beitragen, dass eine Frisur fixiert wird.

Wünschenswert ist nun eine festigende Haar-kosmetische Zubereitung, die die Haare stylt und/oder formt und gleichzeitig einen Schutz vor UV-Strahlen bewirkt.

Überraschenderweise wurde gefunden, dass eine festigende Haar-kosmetische Zubereitung, die ein Octylacrylamide Acrylates Butylaminoethyl Methacrylate Copolymer als festigendes und/oder Film-bildendes Polymer enthält, dem Haar einen UV-Schutz verleiht.

Die Thematik UV-Schutz für Haare ist nicht neu und es finden sich im StdT Dokumente, die sich mit einem UV-Schutz für Haare beschäftigen.

Das Dokument WO 2008/052886 A2 offenbart ein Mittel zur temporären Verformung der Haare. In diesem Mittel muss ein amphoteres Polymer B enthalten sein, das bevorzugt ein Octylacrylamide Acrylates Butylaminoethyl Methacrylate Copolymer ist. Um einen UV-Schutz des Mittels selbst und auch der Haare zu gewährleisten, können dem Mittel bekannte UV-Filter zugesetzt werden.

Die Dokumente DE 10230970 A1 und DE 10352470 A1 offenbaren Haarspray-Zubereitungen, die als festigendes und/oder Film-bildendes Polymer ein Octylacrylamide Acrylates Butylaminoethyl Methacrylate Copolymer enthalten. Um einen UV-Schutz zu gewährleisten, können den Haarsprays bekannte UV-Filter zugesetzt werden.

Aus keinem der Dokumente kann der Fachmann ableiten, dass das festigende und/oder Film-bildende Polymer Octylacrylamide Acrylates Butylaminoethyl Methacrylate Copolymer selbst und allein einen UV-Schutz bewirken kann.

Gegenstand der vorliegenden Erfindung ist also die Verwendung von Octylacrylamide Acrylates Butylaminoethyl Methacrylate Copolymer in einer alkoholischen Lösung zum UV-Schutz der Haare, insbesondere der menschlichen Kopfhaare.

Ebenso Gegenstand der vorliegenden Erfindung ist die Verwendung einer festigenden Haar-kosmetischen Zubereitung, enthaltend Octylacrylamide Acrylates Butylaminoethyl Methacrylate Copolymer in einer alkoholischen Lösung zum UV-Schutz der Haare, insbesondere der menschlichen Kopfhaare.

Der Begriff Haare im Sinne der vorliegenden Erfindung bezieht sich auf menschliche Haare.

Octylacrylamide Acrylates Butylaminoethyl Methacrylate Copolymer ist ein amphoteres festigendes und/oder Film-bildenden Polymer. Octylacrylamide Acrylates Butylaminoethyl Methacrylate Copolymer kann beispielsweise bei der Firma Nouryon unter der Handelsbezeichnung Amphomer erworben werden.

Das Octylacrylamide Acrylates Butylaminoethyl Methacrylate Copolymer enthält Carboxylgruppen. Daher ist eine vollständige oder partielle Neutralisierung vorteilhaft, insbesondere ist eine Neutralisierung von 80 bis 100 % sehr vorteilhaft. Weiterhin ist es bevorzugt, wenn die Neutralisierung mit Aminomethylpropanol (AMP) erfolgt. Andere Neutralisierungsmittel, wie beispielsweise Aminomethylpropandiol, Monoisopropanolamin, Triisopropanolamin und Dimethylstearamin, sind jedoch auch geeignet.

In der festigenden Haar-kosmetischen Zubereitung ist das Octylacrylamide Acrylates Butylaminoethyl Methacrylate Copolymer mit einem Gehalt von 1 bis 6 Gew.-%, bevorzugt von 2 bis 4 Gew.-%, bezogen auf das Gesamtgewicht der Zubereitung, enthalten.

In einer vorteilhaften Ausführungsform ist es bevorzugt, wenn in Bezug auf das festigende und/oder Film-bildende Polymer nur das Octylacrylamide Acrylates Butylaminoethyl Methacrylate Copolymer in der festigenden Haar-kosmetischen Zubereitung enthalten ist.

In einer ebenso vorteilhaften Ausführungsform ist es bevorzugt, wenn in Bezug auf Polymere nur das Octylacrylamide Acrylates Butylaminoethyl Methacrylate Copolymer in der festigenden Haar-Zubereitung enthalten ist.

In einer weiteren ebenso vorteilhaften Ausführungsform ist es bevorzugt, wenn zusätzlich zum Octylacrylamide Acrylates Butylaminoethyl Methacrylate Copolymer ein weiteres festigendes und/oder Film-bildendes Polymer oder mehrere weitere festigende und/oder Film-bildende Polymere enthalten ist/sind, mit der Maßgabe, dass die genannten Polymere Octylacrylamide Acrylates Butylaminoethyl Methacrylate Copolymer nicht umfassen.

Wenn in der erfindungsgemäßen festigenden Haar-kosmetischen Zubereitung weitere festigende und/oder Film-bildende Polymere enthalten sind, so können sie ausgewählt werden aus der Gruppe der alkohollöslichen oder alkohol-dispergierbaren Polyurethane, Polyharnstoffe, Silikonharze und/oder Polyester sowie aus der Gruppe der nichtionischen, anionischen, amphoteren und/oder kationischen festigenden und/oder Film-bildenden Polymere.

Vorteilhafte nichtionische Polymere können beispielsweise aus der Gruppe Vinylpyrrolidon-Homo- oder Copolymerisate ausgewählt werden. Hierzu gehören beispielweise Polyvinylpyrrolidon, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat.

Vorteilhafte anionische Polymere sind beispielsweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere, Natriumpolystyrolsulfonat, Ethylacrylat/N-tert.-Butylacrylamid/Acrylsäure-Copolymere, Vinylpyrrolidon/Vinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere. Weitere bevorzugte anionische Polymere sind Methylvinylether/MaleinsäureCopolymere, die durch Hydrolyse von Vinylether/Maleinsäureanhydrid-Copolymeren entstehen. Diese Polymere können auch teilverestert sein (Ethyl, Isopropyl- bzw. Butylester).

Vorteilhafte amphotere Polymere sind beispielsweise Copolymerisate aus Methacryloylethylbetain und Alkylmethacrylaten vom Typ "Yukaformer", Copolymerisate aus Carboxylgruppen oder Sulfongruppen enthaltenden Monomeren, z.B. (Meth)Acrylsäure und Itaconsäure, mit basischen, insbesondere Aminogruppen enthaltenden Monomeren wie z.B. Mono- bzw. Dialkylaminoalkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure, wobei diese Aufstellung selbstverständlich nicht limitierend sein soll.

Es ist gegebenenfalls vorteilhaft, die anionischen und amphoteren Polymere zur Verbesserung ihrer Wasserlöslichkeit bzw. Treibmittelverträglichkeit mit geeigneten Basen zu neutralisieren. Hierzu können beispielsweise Alkali- und/oder Erdalkalibasen, Ammoniak und/oder verschiedene Amine, wie z.B. Triethanolamin, Triisopropanolamin, Aminomethylpropanol und/oder Aminomethylpropandiol, eingesetzt werden. Die Neutralisation kann je nach Anwendungszweck teilweise oder vollständig erfolgen.

Vorteilhafte kationische Polymere sind beispielsweise Vinylpyrrolidon/Vinylimidazoliummethochlorid-Copolymere, quaternisierte Vinylpyrrolidon/Dialkylaminoalkylmethacrylat-Copolymere, Vinylpyrrolidone/ quaternisiertes Dimethylaminoethylmethacrylate Copolymere (Polyquaternium-11), kationische Cellulose-Derivate, wie z.B. Hydroxyethylcellulose/Dimethylalkylammoniumchlorid-Copolymere sowie Terpolymere aus Vinylcaprolactam/Vinylpyrrolidon mit Dimethylaminoethylmethacrylat bzw. Vinylimmidazoliniummethochlorid und Acrylamidocopolymere, Hydroxyethyl Cellulose Dimethyl Diallylammonium Chloride Copolymer (Polyquaternium-4), Vinylpyrrolidon/ Vinylimidazol/- Methacrylamid/quaternisiertes Vinylimidazol (Polyquaternium-68).

Wenn in der erfindungsgemäßen festigenden Haar-kosmetischen Zubereitung wenigstens ein weiteres festigendes und/oder Film-bildendes Polymer enthalten ist, so liegt dieses Polymer mit einer Gesamtmenge von 0,5 bis 20,0 Gew.-%, bevorzugt 1 bis 10 Gew.%, besonders bevorzugt 1,5 bis 7 Gew.% vor, bezogen auf das Gesamtgewicht der Zubereitungen.

Polymere sind hochmolekulare chemische Verbindungen (Makromoleküle) aus sich wiederholenden Einheiten (Monomere), die gleich oder auch verschieden sein können. Polymere können lineare, verzweigte oder vernetzte Strukturen haben. Eine Klassifizierung der Polymere kann nach ihrer Herkunft erfolgen und zwar in synthetische Polymere, die durch chemische Synthesereaktionen aus Monomeren gebildet werden, natürliche Polymere, die in der Natur gebildet und auch abgebaut werden, und modifizierte natürliche Polymere, die natürliche Polymere sind, die chemisch modifiziert wurden.

Es ist erfindungsgemäß bevorzugt, wenn keine bekannten, üblicherweise eingesetzten UV-Filter in der festigenden Haar-Zubereitung enthalten sind. Dies bedeutet, dass 0 Gew.-% an UV-Filtern in der erfindungsgemäßen festigenden Haar-kosmetischen Zubereitung, bezogen auf das Gesamtgewicht der Zubereitung, enthalten sind.

Üblicherweise werden UV-Filter in chemische UV-Licht absorbierende Substanzen und physikalische "Blocker"-Substanzen eingeteilt, entsprechend ihrem Wirkmechanismus.

Im Allgemeinen sind chemische UV-Licht absorbierende Substanzen (chemische UV-Filter) konjugierte aromatische Verbindungen, die eine Carbonylgruppe aufweisen. Diese Verbindungen absorbieren hochintensive UV-Strahlen, dies ist mit einer Anregung auf einen höheren Energiezustand verbunden. Beim Übergang in den Grundzustand wird die Energie meist in Form von Wärme wieder abgegeben.

Zu der Gruppe der chemischen UV-Licht absorbierenden Substanzen können folgende Substanzgruppen (angegeben jeweils mit der INCI-Bezeichnung) zugeordnet werden: PABA (Aminobenzoesäure), Butyl Methoxydibenzylmethan, Cinoxate, Homosalate, Menthyl Anthranilate, Octocrylene, Octyl Methoxycinnamate, Octyl Triazone, Octyl Salicylate, Benzophenone-3, Octyl Dimethyl PABA, Phenylbenzimidazole Sulfonic Acid, Benzophenone-4.

Physikalische "Blocker"-Substanzen reflektieren und/oder streuen das UV-Licht in der Regel. Nanoskaline physische "Blocker"-Substanzen können auch als UV-Licht-Absorber wirken. Physikalische "Blocker"-Substanzen können als anorganische, partikuläre UV-Filter bezeichnet werden, bekannte Vertreter sind Titandioxid und Zinkoxid.

In der erfindungsgemäßen festigenden Haar-kosmetischen Zubereitung ist Ethanol enthalten, bevorzugt in einer Menge von 10 bis 99 Gew.-%, weiter bevorzugt von 40 bis 95 Gew.-%, bezogen auf das Gesamtgewicht der Zubereitung.

Das Octylacrylamide Acrylates Butylaminoethyl Methacrylate Copolymer ist gut in Ethanol löslich. Da Ethanol eine Verteilung der erfindungsgemäßen Zubereitung in sehr feinen Tröpfchen auf dem Haar ermöglicht und anschließend nach dem Auftragen der erfindungsgemäßen Zubereitung auf die Haare sehr schnell verdunstet, kann ein sehr feiner, gleichmäßiger Film auf den Haaren gebildet werden, der ein überaus befriedigendes Stylen und Fixieren der Frisur erlaubt.

In der festigenden Haar-kosmetischen Zubereitung kann Wasser enthalten sein. Wenn Wasser in der Zubereitung enthalten ist, so liegt Wasser in einer Menge von 0,5 bis 50 Gew.-%, bezogen auf das Gesamtgewicht der Zubereitung, vor.

In der erfindungsgemäßen Zubereitung können zusätzlich zu Ethanol weitere Alkohole enthalten sein.

Alkohole können als Hydroxyl-Derivate von aliphatischen oder alicyclischen, nicht-substituierten Kohlenwasserstoffen definiert werden. Entsprechend der Anzahl der Hydroxylgruppen, die ein Molekül aufweist, können ein-, zwei-, drei- und mehrwertige Alkohole unterschieden werden. Befindet sich die Hydroxylgruppe am primären C-Atom, so werden diese Alkohole primäre Alkohole genannt werden. Einwertige, primäre Alkohole mit 1 bis 3 C-Atomen sind leicht bewegliche Flüssigkeiten, die sich beliebig mit Wasser mischen lassen. Einwertige, primäre Alkohole mit 4 bis 12 C Atomen sind ölige Flüssigkeiten, die nur noch in bestimmten Verhältnissen mit Wasser mischbar sind.

Unter Alkoholen im Sinne der vorliegenden Erfindung werden nur einwertige, primäre Alkohole mit 1 bis 12 C-Atomen verstanden, mit der Maßgabe, dass Ethanol nicht umfasst ist.

Wenn in der erfindungsgemäßen Zubereitung weitere Alkohole enthalten sind, so liegen sie mit einem Gesamtgehalt von 0,1 bis 10 Gew. %, bevorzugt von 1 bis 5 Gew. %, bezogen auf das Gesamtgewicht der Zubereitung, vor.

In der erfindungsgemäßen Zubereitung können zusätzlich ein oder mehrere Emulgator(en) enthalten sein.

Bevorzugte Emulgatoren sind O/W-Emulgatoren. O/W-Emulgatoren zeichnen sich durch HLB-Werte von > 8 bis 15 aus. HLB-Werte lassen sich nach folgender Formel bestimmen:

$$HLB = 20 \times (1- M_{lipophil}/M),$$

wobei $M_{lipophil}$ für die Molmasse des lipophilen Anteils im Emulgator und
M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler bezeichnet.

Folgende Emulgatoren können als O/W-Emulgatoren wirken:
Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Tricetaereth-4 Phosphat, Natriumcetylstearylsulfat, Lecithin Trilaureth-4 Phosphat, Laureth-4 Phosphat, Stearinsäure, Propylenglycolstearat SE, PEG-25- hydriertes Ricinusöl, PEG-54-hydriertes Ricinusöl, PEG-6 Caprylsäure/Caprinsäureglyceride, Glyceryloleat im Gemisch mit Propylenglycol, Ceteth-2, Ceteth-20, Polysorbat 60, Glycerylstearat im Gemisch mit PEG-100 Stearat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol Distearat, PEG-22-Dodecyl Glycol Copolymer, Polyglyceryl-2-PEG-4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45/Dodecylglycol-Copolymer, Methoxy-PEG-22/Dodecylglycol-Copolymer, PEG-20-Glycerylstearat, PEG-20- Glycerylstearat, PEG-8-Bienenwachs, Polyglyceryl-2-lau-rat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Ceteareth-12, Glycerylstearatcitrat, Cetylphosphat, Tricetaereth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth- 10, Oleth-20, Isoceteth-20, Glycerylstearat im Gemisch mit Ceteareth-20, Ceteareth-12, Cetylstearylalkohol und Cetylpalmitat, Cetylstearylalkohol im Gemisch mit PEG-20 Stearat, PEG-30-Stearat, PEG-40-Stearat, PEG-100-Stearat.

Bevorzugt sind diejenigen O/W-Emulgatoren, die sich dadurch auszeichnen, dass sie ethoxylierte Fettalkohole sind oder ethoxylierte Fettalkohole im Gemisch mit einem weiteren Emulgator enthalten. Weiter bevorzugt sind diejenigen ethoxylierten Fettalkohole, deren Alkylrest zum überwiegenden Teil Laurylreste aufweist oder nur Laurylreste aufweist. Besonders bevorzugt sind die Verbindungen Laureth-4, Laureth-5, Laureth-7, Laureth-9, Laureth-10 und Lauerth-12. Ganz besondere bevorzugt ist der Emulgator Laureth-4, der beispielsweise von der Firma Sasol mit der Handelsbezeichnung Marlosol PK 9040 bezogen werden kann.

Wenn in der erfindungsgemäßen Zubereitung ein oder mehrere Emulgator(en) enthalten ist/sind, so liegt/en der oder die Emulgator(en) mit einem Gesamtgehalt von 0,05 bis 2 Gew.-%, bevorzugt 0,1 bis 0,8 Gew.-%, bezogen auf das Gesamtgewicht der Zubereitung und den Aktivgehalt des Emulgators, vor.

In der erfindungsgemäßen Zubereitung können vorteilhaft zusätzlich ausgewählte Parfümrohstoffe enthalten sein. Die Einarbeitung von ausgewählten Parfümrohstoffen dient verschiedenen Zwecken.

Zum einen kann die Zubereitung selbst auf ansprechende Weise parfümiert werden. Dies ist wichtig, weil die Kaufentscheidung und auch die Wiederkaufentscheidung eines Produktes nicht nur durch die eigentlichen Produkteigenschaften bestimmt werden, die oft erst nach längerer Anwendung des Produktes vom Anwender erkannt und geschätzt werden, sondern auch durch ein ansprechendes Parfüm. Ob ein Parfüm gefällt, kann meist gleich zum Kaufzeitpunkt im Laden mit Hilfe eines Testers bestimmt werden.

Zum anderen können Parfümrohstoffe enthalten sein, die vergleichsweise langsam flüchtig sind und dem modellierten Haar einen eigenen Duft verleihen. Auf diese Weise kann der Konsument ein angenehmes Dufterlebnis erfahren, das nicht durch einen starken alkoholischen Geruch überlagert wird.

Die folgenden Parfümkomponenten sind eine Auswahl aus der großen Vielzahl von Parfümrohstoffen, die beispielsweise in die Zubereitung eingearbeitet werden können: Coumarin, Benzylacetat, Benzylalkohol, Benzylbenzoat, Zimtaldehyd, Zimtalkohol, Citral, Citronellol, Eugenol, Geraniol, Limonene, Linalylacetat, Methylbenzoat und Terpineol. Diese Auswahl soll der Verdeutlichung dienen und keineswegs limitierend sein. Weitere Beispiele für Riechstoffe können beispielsweise dem Buch von S. Arctander "Perfume and Flavor Chemicals, Vol I und II, Montclair, N.J., 1969 oder späteren Auflagen oder weiteren Büchern mit vergleichbarem Inhalt entnommen werden oder auch der Patentliteratur auf dem Gebiet der Riechstoffkunde. Diese Literatur ist dem Fachmann auf dem Gebiet der Parfümierung von kosmetischen Produkten bekannt. Die Parfümrohstoffe können in verschiedene Gruppen eingeteilt werden, der Parfümeur würde von Kopfnoten, Mittelnoten und Basisnoten sprechen. Eine derartige Einteilung berücksichtigt beispielsweise die Flüchtigkeit der einzelnen Komponenten. Parfümrohstoffe, die den Kopfnoten zugeordnet werden können, zeichnen sich durch eine größere Flüchtigkeit aus als solche, die den Basisnoten zugeordnet werden. Für die Komponenten der oben genannten Auswahl lassen sich Methylbenzoat, Limonene und Benzylacetat den Kopfnoten zurechnen, während Coumarin, Zimtaldehyd und Zimtalkohol den Basisnoten zugerechnet werden können.

Neben der Flüchtigkeit spielt auch der Geruchseindruck der jeweiligen Parfümrohstoffe eine wichtige Rolle und ist bei der Zusammenstellung einer Parfümmischung zu beachten. Eine Parfümmischung soll immer einen wohlabgestimmten Gesamtgeruch vermitteln.

Wenn in der erfindungsgemäßen Zubereitung Parfümrohstoffe enthalten sind, so liegen diese Parfümrohstoffe liegen als Parfümmischungen vor, und zwar vorteilhaft mit einem Gesamtgehalt von 0,1 bis 1,0 Gew.-%, insbesondere 0,2 bis 0,5 Gew.-%, bezogen auf das Gesamtgewicht der Zubereitung.

In der erfindungsgemäßen Zubereitung können zusätzlich Pflegestoffe enthalten sein. Pflegend in Sinne der vorliegenden Erfindung sind alle Substanzen, die auf dem Haar und auch der Kopfhaut eine pflegende Wirkung erzielen können. Pflegende Wirkung bedeutet im weitesten Sinne eine Oberflächen- und/oder Strukturverbesserung des Haares. Eine pflegende Wirkung im Sinne der vorliegenden Erfindung ist keine dermatologische Wirkung, sondern auf eine kosmetische Wirkung beschränkt. Da die erfindungsgemäße Zubereitung auf dem Haar verbleibt und nicht gleich wieder entfernt wird, können die Pflegestoffe über einen längeren Zeitraum ihre Wirkung entfalten und so zur Haarverbesserung beitragen.

Pflegend im Sinne der vorliegenden Erfindung kann eine Vielzahl von Substanzgruppen wirken, eine Auswahl wird im Folgenden beschrieben, nämlich Substanzen der Vitamine der B Gruppe und/oder des Vitamin B-Komplexes, Silikonverbindungen, polare Öle und/oder Feuchthaltemittel.

In die erfindungsgemäße Zubereitung können zusätzlich eine oder mehrere Substanzen der Vitamine der B Gruppe und/oder des Vitamin B-Komplexes eingearbeitet werden. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in der erfindungsgemäßen Zubereitung.

Panthenol kann beispielsweise unter dem Handelsnamen D-Panthenol 75 W als 77% Lösung in Wasser von der BASF bezogen werden.

Ebenso bevorzugt ist die Verwendung von Niacinamid in der erfindungsgemäßen Zubereitung. Niacinamid kann beispielsweise von der Firma Lonza bezogen werden.

Wenn in der erfindungsgemäßen Zubereitung eine oder mehrere Substanzen der Vitamine der B Gruppe und/oder des Vitamin B-Komplexes enthalten ist/sind, so liegt/en die eine oder mehrere Substanzen der Vitamine der B Gruppe und/oder des Vitamin B-Komplex mit einem Gesamtgwicht von 0,005 Gew.-% bis 5 Gew.-%, bevorzugt 0,05 Gew.-% bis 2 Gew.-%, insbesondere bevorzugt 0,01 Gew.-% bis 1,0 Gew.-% vor, bezogen auf das Gesamtgewicht der Zubereitung und den Aktivgehalt der jeweiligen Substanz, in der erfindungsgemäßen Zubereitung enthalten.

Erfindungsgemäß möglich ist ebenfalls, wenn zusätzlich eine oder mehrere Silikonverbindungen in die erfindungsgemäße Zubereitung eingearbeitet werden. Silikone haben auch die chemische Bezeichnung Poly(organo)siloxane. Es handelt sich um eine Gruppe synthetischer Polymere, bei denen Siliziumatome über Sauerstoffatome verknüpft sind. Silikone bestehen aus einzelnen Siloxaneinheiten, die aus Siliciumatomen, verbunden über eine Sauerstoffbrücke, bestehen.

Die allgemeine Formel lautet $R_nSiO_{(4-n)/2}$ (n=0, 1, 2, 3), d. h. eine Siloxaneinheit kann ein bis vier weitere Substituenten aufweisen.

Siloxaneinheiten können also mono-, di-, tri- und tetrafunktionell sein. In symbolischer Schreibweise stellt man dies durch folgende Buchstaben dar:

M (mono), M entspricht $R_3SiO_{1/2}$,
D (di), D entspricht $R_2SiO_{2/2}$,
T (tri), T entspricht $RSiO_{3/2}$ und
Q (quatro), Q entspricht $SiO_{4/2}$.

Wie bei den organischen Polymeren basiert die Vielzahl der möglichen Verbindungen darauf, dass verschiedene Siloxaneinheiten im Molekül miteinander verknüpft werden können. Angelehnt an die Systematik der organischen Polymere kann man folgende Gruppen unterscheiden:

- Cyclische Polysiloxane sind ringförmig aus difunktionellen Siloxaneinheiten aufgebaut, $D_n$.
- Lineare Polysiloxane sind folgendermaßen aufgebaut $MD_nM$, bzw. $R_3SiO[R_2SiO]_nSiR_3$, als Beispiel sei Polydimethylsiloxan genannt.
- Vernetzte Polysiloxane sind ketten- oder ringförmige Moleküle, die mit Hilfe von tri- und tetrafunktionellen Siloxaneinheiten zu ebenen oder dreidimensionalen Netzwerken verknüpft sind. Der Aufbau hochmolekularer Silikone erfolgt über Kettenbildung und Vernetzung.
- Verzweigte Polysiloxane weisen als verzweigende Elemente trifunktionelle oder tetrafunktionelle Siloxaneinheiten auf, $M_nD_mT_n$. Die Verzweigungsstellen sind dabei entweder in eine Kette oder einen Ring eingebaut.

An den Siliciumatomen der Silikone können verschiedene Substituenten gebunden sein. Sehr häufig sind dies Methylgruppen, entsprechend können lineare, verzweigte oder vernetzte Polydimethylsiloxane unterschieden werden. Je nach Länge und Verzweigungs- bzw. Vernetzungsgrad entstehen verschieden große und komplexe Moleküle. Bei flüssigen oder fließfähigen Polydimethylsiloxanen erfolgt eine Charakterisierung häufig mithilfe von entsprechenden Viskositätswerten, die der Hersteller oder Lieferant angibt.

Neben den Methylgruppen als Substituenten gibt es eine Vielzahl anderer Substituenten. Um die Löslichkeit der Polysiloxane zu verbessern und damit bessere Einarbeitungsmöglichkeiten in wässrige kosmetische Zubereitungen zu, können AlkoxyReste, insbesondere Ethylenoxidreste in die Polysiloxane eingeführt werden. Ethylenoxidreste werden auch als Polyethylenglycolreste bezeichnet, mit der Abkürzung PEG.

Erfindungsgemäß besonders vorteilhaft ist es Polysiloxane einzusetzen, die 8 bis 20 Ethylenoxidreste, bevorzugt 10 bis 18 Ethylenoxidreste, aufweisen. Ein bevorzugtes Beispiel einer derartigen Polysiloxanverbindung ist PEG-12 Dimethicone, das beispielsweise von der Firma Dow Corning mit der Handelsbezeichnung Xiameter OFX-0193 Fluid erworben werden kann.

Wenn in der erfindungsgemäßen Zubereitung ein oder mehrere Polysiloxanverbindung(en), optional aufweisend 8 bis 20 Ethylenoxidreste, bevorzugt 10 bis 18 Ethylenoxidreste enthalten ist/sind, so liegt/en sie mit einem Gesamtgehalt von 0,01 bis 3 Gew.-%, bevorzugt 0,05 bis 1,0 Gew.-% in der Zubereitung vor, bezogen auf das Gesamtgewicht der Zubereitung und den Aktivgehalt der genannten Polysiloxanverbindungen.

In die erfindungsgemäße Zubereitung können zusätzlich ein oder mehrere Öle eingearbeitet werden. Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weiterhin vorteilhaft ist Squalen, eine mehrfach ungesättigte organische Verbindung aus der Gruppe der Triterpene.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodecey-Imyristat, Octyldodekanol, Cetearylisononanoat,Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, E-rucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC erhältlich ist.

Auch beliebige Abmischungen der genannten Ölkomponenten sind vorteilhaft im Sinne der vorliegenden Erfindung einzusetzen.

Besonders bevorzugt sind Öle wie Sonnenblumenöl, Rizinusöl, Sojaöl, Jojobaöl und Macadamiaöl, die als pflegende Öle bezeichnet werden können. Ebenso besonders bevorzugt ist Squalen.

Wenn in der erfindungsgemäßen Zubereitung ein oder mehrere Öle enthalten ist/sind, so liegen sie mit einem Gesamtgehalt von 0,01 bis 1,0 Gew.-%, bevorzugt 0,03 bis 0,1 Gew.-%, bezogen auf das Gesamtgewicht der Zubereitung, vor.

In die erfindungsgemäße Zubereitung können zusätzlich ein oder mehrere Feuchthaltemittel eingearbeitet werden. Feuchthaltemittel können beispielsweise den Haaren Feuchtigkeit verleihen und/oder dazu beitragen, einen Feuchtigkeitsverlust der Haare zu verhindern. Bei den Feuchthaltemitteln handelt es sich um hygroskopische Substanzen, die Wasser binden können. Dieses Wasserbindevermögen beruht auf hydrophilen Gruppen, wie Hydroxylgruppen, aber auch Amingruppen und Carboxylgruppen. Feuchthaltemittel sind beispielsweise Propylenglycol, Hexylenglycol, Butylenglycol, Glyceryltriacetat, Glycerin, Sorbitol, Xylitol, Maltitol, Polydextrose, Harnstoff, Aloe Vera Gel, Honig und alpha-Hydroxysäuren, wie beispielsweise Milchsäure. Erfindungsgemäß bevorzugt ist der Einsatz von Propylenglycol und/oder Glycerin.

Wenn in der erfindungsgemäßen Zubereitung ein Feuchthaltemittel oder mehrere Feuchthaltemittel enthalten ist/sind, so liegen sie mit einem Gesamtgehalt von 0,01 bis 1 Gew.-%, bevorzugt 0,1 bis 0,8 Gew.-% bezogen auf das Gesamtgewicht der Zubereitung, vor.

In die erfindungsgemäße Zubereitung können zusätzlich ein oder mehrere Antioxidantien enthalten. Antioxidantien sind Moleküle, die verhindern, dass andere Moleküle oxidiert werden. In Oxidationsreaktionen können freie Radikale entstehen, die wiederum andere Moleküle schädigen oder zerstören. Die freien Radikale können beispielsweise durch Antioxidantien unschädlich gemacht werden. Zu den Antioxidantien können verschiedene Moleküle gezählt werden, die natürlichen oder synthetischen Ursprungs sind. Unter den synthetischen Molekülen können Verbindungen wie Butylhydroanisol (BHA), Butylhydroxytoluol (BHT) und Pentaerythrityl tetra-di-t-butyl-Hydroxyhydrocinnamat angeführt werden, erfindungsgemäß bevorzugt ist Butylhydroxytoluol.

Als Antioxidans natürlichen Ursprung sei γ-Oryzanol genannt. Bei γ-Oryzanol handelt es sich um Ferulasäureester von Phytosterolen.

Wenn in der erfindungsgemäßen Zubereitung wenigstens ein Antioxidans enthalten ist, so liegt es mit einem Gesamtgehalt von 0,01 bis 1,0 Gew.-%, bevorzugt von 0,02 bis 0,7 Gew.-% vor, bezogen auf das Gesamtgewicht Zubereitung und den Aktivgehalt des wenigstens einen Antioxidans.

Der erfindungsgemäßen, festigenden Haar-kosmetischen Zubereitung kann ein Treibmittel zugesetzt werden. Das Treibmittel kann aus einem oder mehreren Treibgasen bestehen, das/die ausgewählt werden kann/können aus Kohlenwasserstoffgasen, Dimethylether, Stickstoff, Luft oder Kohlendioxid oder Mischungen davon.

Eine vorteilhafte Ausführungsform ist dadurch charakterisiert, dass das Treibmittel ein oder mehrere druckverflüssigte(s) Treibgas(e) ist/sind. Hierbei ist es bevorzugt, wenn die Treibgase ausgewählt werden aus Kohlenwasserstoffen oder Dimethylether, insbesondere eine Mischung aus Isobutan, Propan und n-Butan oder Dimethylether.

Wenn der erfindungsgemäßen Zubereitung ein oder mehrere druckverflüssigte(s) Treibgas(e) zugesetzt werden, so liegt/en es/sie mit einem Gesamtgewicht von 30 bis 40 Gew.-% vor, bezogen auf das Gesamtgewicht der Zubereitung.

Die Druckgasstufe wird vorteilhaft aus dem Bereich von 2,0 bis 3,5 bar ausgewählt.

Eine weitere, genauso vorteilhafte Ausführungsform ist dadurch charakterisiert, dass das Treibmittel aus einem oder mehreren komprimierten Gase ausgewählt wird. Hierbei ist es bevorzugt, wenn die Gase ausgewählt werden aus Luft, Stickstoff, Kohlendioxid oder Mischungen davon, besonders bevorzugt ist Stickstoff.

Wenn der erfindungsgemäßen Zubereitung ein oder mehrere komprimierte(s) Gas(e) zugesetzt wird/werden, so liegt/en es/sie mit einem Gesamtgewicht 30 bis 70 Gew.-%, bevorzugt 40 bis 50 Gew.-% vor, bezogen auf das Gesamtgewicht der Zubereitung.

Der Fülldruck des Gases wird über ein Manometer geregelt; der Fülldruck beträgt 8 bis12 bar.

Eine ebenso vorteilhafte Ausführungsform ist dadurch charakterisiert, dass die erfindungsgemäße, festigende Haar-kosmetischen Zubereitung aus einem Treibgas-freien, mechanisch zu bedienenden Pumpzerstäuber (Pumpspender) entnommen wird.

Erfindungsgemäße Zubereitungen gemäß Ausführungsformen, die ein oder mehrere Treibgase enthalten, sind in Aerosol-Verpackungseinheiten enthalten.

Eine Aerosol-Verpackungseinheit ist ein Druckgasbehälter, der eine Dose und eine Abgabevorrichtung aufweist. Die Aerosol-Verpackungseinheit steht unter Druck von dem Treibmittel.

Die erfindungsgemäßen Dosen können im Wesentlichen zylindrische Gefäße aus Metall (Aluminium, Weißblech, Inhalt <1000 ml) sein, bei deren Auswahl Druck- und Bruchfestigkeit, Korrosionsbeständigkeit, leichte Füllbarkeit, usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 15 bar und das maximale Füllvolumen bei dieser Temperatur ca. 90 % des Gesamtvolumens.

Der innere Aufbau der Dosen sowie die Ventilkonstruktion sind je nach Verwendungs-Zweck und der physikalischen Beschaffenheit des Inhalts - z. B. ob als Ein-, Zwei- oder als Dreiphasensystem - sehr variantenreich und können vom Fachmann durch einfaches Ausprobieren ohne erfinderisches Zutun ermittelt werden. Für geeignete Ausführungsformen sei auf das "Aerosol Technologie Handbuch der Aerosol-Verpackung" hingewiesen (Wolfgang Tauscher, Melcher Verlag GmbH Heidelberg/München, 1996).

Ventile können mit oder ohne Steigrohr ausgebildet sein. Die Einzelteile, aus welchen Ventile üblicherweise aufgebaut sind, bestehen meist aus den folgenden Materialien:

| | |
|---|---|
| Teller: | Weißblech: blank, gold- bzw. klarlackiert, folienkaschiert (PE, PP oder PET) |
| | Aluminium: blank, silber- oder goldlackiert, verschiedene Lackvarianten, Stoner-Mudge-Ausführung. |
| Dichtung: | natürliche bzw. synthetische Elastomere bzw. thermoplastische (Sleeve-Gaskets, folienkaschiert aus PE oder PP) Innen- und Außendichtungen, z. B. aus Perbunan, Buna, Neopren, Butyl, CLB, LDPE, Viton, EPDM, Chlorbutyl, Brombutyl und/oder diversen Compounds. |
| Kegel: | Polyamid (PA), Polyoxymethylen (POM), Messing sowie diversen Sondermaterialen, Standardbohrungen, beispielsweise die Zentralbohrung (z. B.: 0,25 bis 0,70 mm oder 2 x 0,45 bis 2 x 1,00 mm), verschiedene Schaftdurchmesser. |
| Feder: | Metall, besonders bevorzugt V2A, rostfreier Stahl; Kunststoff und auch Elastomer. |
| Gehäuse: | Standard und Impact, |
| | Gasphasen-Bohrungen, RPT-Bohrungen |
| | Materialien: z. B. Polyacetal, PA, PE, POM und dergleichen mehr. |
| Steigrohr: | Kunststoff (Polymer Resin), z.B. PE, PP, PA oder Polycarbonat. |

Die Zubereitung gemäß der Ausführungsform, die dadurch gekennzeichnet ist, dass das Treibmittel ein oder mehrere druckverflüssigte(s) Treibgas(e) ist/sind, wird in einen geeigneten Aerosolbehälter abgefüllt. Unter dem Aerosolprodukt wird die erfindungsgemäße Zubereitung und das Packmittel verstanden. Ein Packmittel für das Aerosolprodukt muss für die genannte Ausführungsform geeignet sein. Geeignete Dosen, beispielsweise aus Aluminium oder Weißbleich, sind beispielsweise bei den Firmen Tubex, Ball Packaging Europe oder Nussbaum erhältlich. Geeignete Ventile können beispielsweise von den Firmen Deutsche Präzisions-Ventil GmbH, Aptar oder Lindal erhalten werden.

Die Zubereitung gemäß der Ausführungsform, die dadurch gekennzeichnetist, dass das Treibmittel aus einem oder mehreren komprimierten Gase ausgewählt wird, wird in einen geeigneten Aerosolbehälter abgefüllt. Von den oben genannten Firmen sind auch Dosen oder Ventile für die hier genannte Ausführungsform erhältlich.

Die erfindungsgemäße, festigende Haar-kosmetische Zubereitung gemäß der Ausführungsform, die dadurch gekennzeichnetist, dass die erfindungsgemäße Zubereitung aus einem Treibgas-freien, mechanisch zu bedienenden Pumpzerstäuber (Pumpspender) entnommen wird, wird in einen geeigneten Pumpspender abgefüllt.

Um einen Nachweis für einen UV-Schutz von Haaren durch das Polymer Octylacrylamide Acrylates Butylaminoethyl Methacrylate Copolymer zu erhalten, wurde eine High Pressure Differential Scanning Calorimetry (HP-DSC) Messung durchgeführt.

Das Säugetier-Haar besteht hauptsächlich aus $\alpha$-Keratin, das in einer rechtsgängigen $\alpha$-Helix vorliegt. Das $\alpha$-Keratin weist eine große Anzahl an hydrophoben Aminosäuren auf (Phenylalanin, Isoleucin, Valin, Methionin und Alanin). Zwei $\alpha$-Helices des $\alpha$-Keratins sind umeinandergewunden und bilden eine Super-Helix. Kovalente Quervernetzungen über Disulfid-Brücken stabilisieren diese Superhelix, die auch als Protofilament bezeichnet wird.

Bei einer HP-DSC-Messung durchgeführt mit menschlichem Haar, wird die thermische Stabilität der wichtigsten morphologischen Komponenten des Haares bestimmt. Diese Komponenten können als teilweise helikale, faserige Zwischenfilamente und Proteine, die mit den Zwischenfilament assoziiert sind und eine vernetzte, amorphe Matrix bilden, identifiziert werden. Eine HP-DSC-Messung liefert die Denaturierungs-Temperatur und die Denaturierungs-Enthalpie; der jeweilige Wert hängt vom Ausmaß der strukturellen Integrität des $\alpha$-helikalen Materials und von der Vernetzungsdichte der Matrix ab.

Eine Schädigung des $\alpha$-Keratins kann durch äußere Einflüsse, die auf das Haar einwirken, geschehen. Dies kann sowohl Hitze als auch UV-Strahlung sein. Geschädigtes $\alpha$-Keratin denaturiert schneller, d.h. die Denaturierungstemperatur ist geringer. Wenn nun eine Substanz auf geschädigtes Haar aufgetragen wird und den Denaturierungsprozess verzögert, d.h. die Denaturierungstemperatur liegt höher als bei unbehandeltem geschädigtem Haar, so ist gezeigt, dass diese Substanz bewirkt, dass das $\alpha$-Keratin und die amorphe Matrix geschützt ist. Dieser Schutz durch die jeweilige Substanz ist nun nicht nur bei einer HP-DSC-Messung wirksam, sondern auch bei äußeren Einflüssen, die auf das Haar einwirken, also auch bei UV-Licht.

Folgende Zubereitung wurde hergestellt (die Mengenangaben sind in Gew.-% angegeben):

| INCI | Beispiel A (Code 10) |
|---|---|
| Panthenol | 0,11 |
| Octylacrylamide Acrylates Butylaminoethyl Methacrylate Copolymer | 3,67 |
| Parfüm | 0,35 |
| Aminopropanol | 0,66 |

(fortgesetzt)

| INCI | Beispiel A (Code 10) |
|---|---|
| Alkohol denat. | Ad 100 |
| Wasser | 0,03 |
| Propan/Butan/Isobutan-Gemisch | 35,0 |

Die Messung erfolgte an Haarsträhnen (europäisches, ungeschädigtes Haar, jeweils 2 g). Die Haarsträhnen (Code 01, 02 und 10) wurden 15 Minuten in Wasser angefeuchtet und anschließend mit einem Beiersdorf-Standard-Haarschampoo (Zusammensetzung siehe Tabelle) gewaschen.

| Inhaltsstoff | [Gew.-%] |
|---|---|
| Laurylethersulfat | 12,5 |
| Natriumchlorid | 1,0 |
| Natriumbenzoat | 0,4 |
| Citronensäure | 0,1 |
| Wasser | 86,0 |

Die Probe mit dem Code 01 für 18 Stunden in einem klimatisierten Raum (22 ±1°C, 55 ±5% relative Luftfeuchtigkeit) getrocknet (unbehandelte und ungeschädigte Probe).

Bei der Probe mit dem Code 02 wurde nach dem Waschen das Wasser ausgepresst, dann erfolgte eine UV-Licht-Behandlung für 24 Stunden (Atlas Suntester XLS, 765 W/m, 35°C). Dann wurde die Probe für 18 Stunden in einem klimatisierten Raum (22 ±1°C, 55 ±5% relative Luftfeuchtigkeit) gelagert (unbehandelte und UV-geschädigte Probe).

Bei der Probe mit dem Code 10 wurde nach dem Waschen das Wasser ausgepresst und dann wurde die Zubereitung gemäß Beispiel A mit drei Sprühstößen aus 20 cm Abstand vorn und hinten auf die Haarprobe aufgetragen. Anschließend erfolgte die UV-Behandlung wie für die Probe mit dem Code 02 angegeben. Dann wurde die Probe für 18 Stunden in einem klimatisierten Raum (22 ±1°C, 55 ±5% relative Luftfeuchtigkeit) gelagert (behandelte, UV-geschädigte Probe).

Jede einzelne Haarsträhne wurde zerkleinert (etwa 1 mm lange Stücke) und zwischen 9 mg und 12 mg Haarmaterial in einen Tiegel aus Edelstahl (Large Volume Capsules (LVC), Perkin Elmer Part Number: 0319 0218) überführt. Nach Zugabe von 50 $\mu$l demineralisiertem Wasser wurden die Tiegel mit Dichtungsring hermetisch verschlossen und über Nacht bei Raumtemperatur gelagert. Dann erfolgte die HP-DSC-Messung, die für jede Haarsträhne 12mal wiederholt wurde.

Die Messungen erfolgten mit dem Gerät PerkinElmer Pyris 1 DSC, die Software für die Datenanalyse war der Pyris Manager, die Messung erfolgte im Temperaturbereich von 50 - 180°C, die Heizrate betrug 10°C/min.

Folgende Proben (Haarsträhnen) wurden vermessen:

| Probennummer | Art der Probe | Bemerkung |
|---|---|---|
| 01 | Unbehandelte Kontrolle | nicht geschädigt |
| 02 | Unbehandelt | UV geschädigt |
| 10 | Behandelt mit Beispielzubereitung A | UV geschädigt |

Messergebnisse:

|    | Peak-Temperatur | |
|----|-----------|---------|
|    | Mittelwert | Standardabweichung |
| 01 | 157,18 | 0,57 |
| 02 | 153,57 | 0,26 |
| 10 | 154,74 | 0,22 |

Die Behandlung der Haarsträhne mit der Beispielzubereitung A (Probe mit dem Code 10; behandelt + UV-geschädigt) führte dazu, dass die Denaturierungstemperatur der untersuchten Probe höher lag als bei der Probe, die nicht mit dieser Zubereitung behandelt worden war (Probe mit dem Code 02; unbehandelt + UV-geschädigt). Der Unterschied ist signifikant.

Damit ist belegt, dass das Octylacrylamide Acrylates Butylaminoethyl Methacrylate Copolymer bewirkt, dass Haare, die damit behandelt werden, einen verbesserten UV-Schutz erhalten.

**Beispiele:**

Nachfolgende Beispiele sollen die Erfindung verdeutlichen, ohne sie einzuschränken. Die Mengenangaben sind Angaben in Gew.-% und beziehen sich auf den jeweiligen Aktivgehalt.

| INCI | 1 | 2 | 3 | 4 | 5 | 6 |
|------|---|---|---|---|---|---|
| Panthenol | 0,4 | 0,15 | 0,1 | 0,15 | 0,1 | 0,15 |
| Niacinamide | 0,03 | 0,03 | 0,03 | 0,03 | 0,01 | 0,03 |
| Oryzanol | | 0,023 | | | 0,01 | |
| Isopropyl Myristate | 0,1 | 0,2 | | | 0,7 | |
| Octylacrylamide Acrylates Butylaminoethyl Methacrylate Copolymer | 3,3 | 3,3 | 3,7 | 2,5 | 1,5 | 3,8 |
| Parfum | 0,5 | 0,35 | 0,4 | 0,35 | 0,35 | 0,35 |
| Aminomethyl Propanol | 0,6 | 0,6 | 0,68 | 0,4 | 0,25 | 0,69 |
| Alcohol Denat. | ad 100 | ad 100 | ad 100 | ad 100 | ad 100 | ad 100 |
| Propane/Butane/Isobutane-Gemisch | 40 | 35 | 30 | 35 | | |
| Nitrogen | | | | | 50 | |

**Patentansprüche**

1. Verwendung von Octylacrylamide Acrylates Butylaminoethyl Methacrylate Copolymer in einer alkoholischen Lösung zum UV-Schutz der Haare, insbesondere Kopfhaare.

2. Verwendung einer festigenden Haar-kosmetischen Zubereitung, enthaltend Octylacrylamide Acrylates Butylaminoethyl Methacrylate Copolymer in einer alkoholischen Lösung zum UV-Schutz der Kopfhaare.

3. Verwendung nach Anspruch 1 oder 2, **dadurch gekennzeichnet, dass** Octylacrylamide Acrylates Butylaminoethyl Methacrylate Copolymer partiell oder vollständige neutralisiert vorliegt, bevorzugt zu 80 bis 100 % neutralisiert.

4. Verwendung nach Anspruch 1 oder 2 und/oder 3, **dadurch gekennzeichnet, dass** als Neutralisierungsmittel Aminomethylpropanol, Aminomethylpropandiol, Monoisopropanolamin, Triisopropanolamin und/oder Dimethylstearamin gewählt werden, bevorzugt Aminomethylpropandiol.

5. Verwendung nach Anspruch 1 oder wenigstens einem der Ansprüche 2 bis 4, **dadurch gekennzeichnet, dass** Octylacrylamide Acrylates Butylaminoethyl Methacrylate Copolymer mit einem Gehalt von 1 bis 6 Gew.-%, bevorzugt von 2 bis 4 Gew.-%, bezogen auf das Gesamtgewicht der Zubereitung, enthalten ist.

6.  Verwendung nach Anspruch 1 oder wenigstens einem der Ansprüche 2 bis 5, **dadurch gekennzeichnet, dass** als festigendes und/oder Film-bildendes Polymer, nur das Octylacrylamide Acrylates Butylaminoethyl Methacrylate Copolymer enthalten ist.

7.  Verwendung nach Anspruch 1 oder wenigstens einem der Ansprüche 2 bis 6, **dadurch gekennzeichnet, dass** als Polymer nur das Octylacrylamide Acrylates Butylaminoethyl Methacrylate Copolymer enthalten ist.

8.  Verwendung nach Anspruch 1 oder wenigstens einem der Ansprüche 2 bis 5, **dadurch gekennzeichnet, dass** zusätzlich zum Octylacrylamide Acrylates Butylaminoethyl Methacrylate Copolymer ein weiteres festigendes und/oder Film-bildendes Polymer oder mehrere weitere festigende und/oder Film-bildende Polymere enthalten ist/sind, wobei das oder genannten weiteren festigenden und/oder Film-bildenden Polymere aus der Gruppe der alkohollöslichen oder alkohol-dispergierbaren Polyurethane, Polyharnstoffe, Silikonharze und/oder Polyester sowie aus der Gruppe der nichtionischen, anionischen, amphoteren und/oder kationischen festigenden und/oder Film-bildenden Polymere ausgewählt werden,
    mit der Maßgabe, dass die genannten Polymere Octylacrylamide Acrylates Butylaminoethyl Methacrylate Copolymer nicht umfassen.

9.  Verwendung nach Anspruch 1 oder wenigstens einem der Ansprüche 2 bis 8, **dadurch gekennzeichnet, dass** die Zubereitung keine UV-Filter enthält, die aus UV Licht absorbierenden organisch-chemischen Substanzen und/oder anorganischen, partikulären UV-Filtern ausgewählt werden.

10. Verwendung nach Anspruch 1 oder wenigstens einem der Ansprüche 2 bis 9, **dadurch gekennzeichnet, dass** Ethanol in einer Menge von 10 bis 99 Gew.%, bevorzugt von 40 bis 95 Gew.-%, bezogen auf das Gesamtgewicht der Zubereitung, enthalten ist.

11. Verwendung nach Anspruch 1 oder wenigstens einem der Ansprüche 2 bis 10, **dadurch gekennzeichnet, dass** Wasser in einer Menge von 0,5 bis 50 Gew. %, bezogen auf das Gesamtgewicht der Zubereitung, enthalten ist.

12. Verwendung nach Anspruch 1 oder wenigstens einem der Ansprüche 2 bis 11, **dadurch gekennzeichnet, dass** zusätzlich ausgewählte Parfümrohstoffe enthalten sind, bevorzugt mit einem Gehalt von 0,1 bis 1,0 Gew.-%, insbesondere 0,2 bis 0,5 Gew.-%, bezogen auf das Gesamtgewicht der Zubereitung.

13. Verwendung nach wenigstens einem der Ansprüche 2 bis 12, **dadurch gekennzeichnet, dass** ein Treibmittel, ausgewählt aus einem oder mehreren druckverflüssigten Treibgasen, bevorzugt ausgewählt aus Kohlenwasserstoffen und Dimethylether, insbesondere bevorzugt aus einer Mischung aus Isobutan, Propan und n-Butan oder Dimethylether, enthalten ist.

14. Verwendung nach wenigstens einem der Ansprüche 2 bis 12, **dadurch gekennzeichnet, dass** ein Treibmittel, ausgewählt aus einem oder mehreren komprimierten Gasen, bevorzugt ausgewählt aus Luft, Stickstoff, Kohlendioxid oder Mischungen davon, insbesondere bevorzugt Stickstoff, enthalten ist.

15. Verwendung nach wenigstens einem der Ansprüche 2 bis 12, **dadurch gekennzeichnet, dass** die erfindungsgemäße Zubereitung aus einem Treibgas-freien, mechanisch zu bedienenden Pumpzerstäuber (Pumpspender) entnommen wird.

Europäisches
Patentamt

European
Patent Office

Office européen
des brevets

# EUROPÄISCHER RECHERCHENBERICHT

**Nummer der Anmeldung**

EP 23 15 4190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | DE 102 30 970 A1 (BEIERSDORF AG [DE]) 22. Januar 2004 (2004-01-22) * das ganze Dokument * ----- | 1 | INV. A61K8/81 A61Q5/00 A61Q5/06 |
| X,P | Nouryon: "Perfect Party Hair with Nouryon", , 24. Mai 2023 (2023-05-24), XP093074997, Gefunden im Internet: URL:https://www.youtube.com/watch?v=h8y9os vlNRE [gefunden am 2023-08-19] * das ganze Dokument * ----- | 1 | |

**RECHERCHIERTE SACHGEBIETE (IPC)**

A61K
A61Q

Der vorliegende Recherchenbericht wurde für alle Patentansprüche erstellt

| Recherchenort | Abschlußdatum der Recherche | Prüfer |
|---|---|---|
| München | 25. August 2023 | Skulj, Primoz |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

EP 4 265 238 A1

**ANHANG ZUM EUROPÄISCHEN RECHERCHENBERICHT**
**ÜBER DIE EUROPÄISCHE PATENTANMELDUNG NR.**

EP 23 15 4190

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-08-2023

| Im Recherchenbericht angeführtes Patentdokument | Datum der Veröffentlichung | Mitglied(er) der Patentfamilie | Datum der Veröffentlichung |
|---|---|---|---|
| DE 10230970 A1 | 22-01-2004 | KEINE | |

Für nähere Einzelheiten zu diesem Anhang : siehe Amtsblatt des Europäischen Patentamts, Nr.12/82

EPO FORM P0461

14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008052886 A2
- DE 10230970 A1
- DE 10352470 A1

**In der Beschreibung aufgeführte Nicht-Patentliteratur**

- **BUCH VON S. ARCTANDER.** Perfume and Flavor Chemicals. Montclair, 1969, vol. I,II
- **WOLFGANG TAUSCHER.** Aerosol Technologie Handbuch der Aerosol-Verpackung. Melcher Verlag GmbH, 1996